# EUROPÄISCHE PATENTSCHRIFT

(11) **EP 1 993 489 B1**
(45) Veröffentlichungstag und Bekanntmachung des Hinweises auf die Patenterteilung: **17.02.2010**
(21) Anmeldenummer: 07726814.2
(22) Anmeldetag: 12.03.2007
(51) Int. Cl.: A61F 5/00, A61B 17/135

(54) **ADAPTIVE EINRICHTUNG ZUR AUTOMATISIERTEN ANPASSUNG DER MAGENÖFFNUNG EINES PATIENTEN**
ADAPTIVE DEVICE FOR AUTOMATICALLY ADAPTING THE STOMACH OPENING OF A PATIENT
DISPOSITIF ADAPTATIF POUR ADAPTER L'OUVERTURE DE L'ESTOMAC D'UN PATIENT DE FAÇON AUTOMATISÉE

(30) Priorität: 13.03.2006 WO PCT/EP2006/060667
(43) Veröffentlichungstag der Anmeldung: 26.11.2008
(73) Patentinhaber: Steffen, Rudolf, 3011 Bern (CH)
(72) Erfinder: Steffen, Rudolf, 3011 Bern (CH)
(74) Vertreter: Scheuzger, Beat Otto
(86) Internationale Anmeldenummer: PCT/EP2007/052308
(87) Internationale Veröffentlichungsnummer: WO 2007/104745

(56) Entgegenhaltungen:
- EP-A- 1 600 128
- WO-A-00/09049
- WO-A-2004/012806
- US-A- 5 938 669

## Beschreibung

### Technisches Gebiet

Die Erfindung betrifft eine adaptive Einrichtung zur automatisierten Anpassung der Magenöffnung eines Patienten, umfassend ein Magenband mit einem nicht dehnbaren Rückenteil und einer ersten dehnbaren Kammer, und eine mit der ersten dehnbaren Kammer verbundene zweite dehnbare Kammer. Die Erfindung betrifft insbesondere eine adaptive Einrichtung zur automatisierten Anpassung der Magenöffnung eines Patienten, in welcher das Magenband zur Anpassung der Magenöffnung des Patienten um den Magen des Patienten herum platzierbar ist, und die Flüssigkeit mittels einer Beförderungsvorrichtung aus der einen dehnbaren Kammer in die andere dehnbare Kammer verschiebbar ist.

### Stand der Technik

Krankhafte Fettleibigkeit oder Adipositas ist ein immer grösseres Problem in der heutigen modernen Gesellschaft. Krankhaft fettleibige Patienten neigen stark zu diversen so genannten Folgekrankheiten, von welchen insbesondere Herz-Kreislauf-Erkrankungen wie Bluthochdruck, Stoffwechselstörungen wie Diabetes oder Gicht und psychische Störungen wie Depressionen, aber auch übermässiger Gelenkenverschleiss oder erhöhtes Hirnschlagrisiko eine wichtige Rolle spielen. In diesem Zusammenhang hat die krankhafte Fettleibigkeit auch äusserst negative Auswirkung auf die Volkswirtschaft der Länder, da die Ausgaben sowohl für die direkten Kosten (für die ärztliche und stationäre Behandlung der Krankheit, sowie die entsprechenden Medikamente), als auch für die indirekten Kosten (Kosten für die Behandlung der Folgekrankheiten) der Fettleibigkeit in gewissen Ländern fast 10% der Gesamtkosten des Gesundheitssystems ausmachen.

Es sind auch verschiedene chirurgische Verfahren bekannt, welche zur Behandlung der krankhaften Fettleibigkeit eingesetzt werden. So wird beispielsweise bei einem so genannten operativen Bypass eine direkte Verbindung zwischen dem Magen und dem Dünndarm hergestellt. In diesem Fall durchläuft ein Teil der aufgenommenen Nahrung nicht den gesamten Verdauungstrakt, so dass auch deren Verwertung im Körper einen viel niedrigeren Grad aufweist. In einem anderen chirurgischen Verfahren werden in der Magenwand vertikale oder horizontale Nähte oder Klammern angebracht, so dass das Volumen des Magens reduziert wird, womit auch die Menge bzw. die Geschwindigkeit des Durchgangs der aufgenommenen Nahrungsmittel verkleinert wird. Die Nachteile dieser chirurgischen Verfahren liegen insbesondere darin, dass es dabei um vollständige operative Eingriffe ins Gewebe des Patienten handelt, welche für den Patienten sehr traumatisierend sind. Ausserdem treten bei solchen chirurgischen Eingriffen Komplikationen sowohl vor, als auch während und nach der Operation nicht selten auf, so dass die Gesundheit des Patienten noch weiter verschlechtert werden könnte.

Bei einem anderen Verfahren wird ins Mageninnere des Patienten ein aufblasbarer Ballon (in der Regel aus weichem Kunststoff) eingeführt. Dabei kann der Ballon entweder operativ oder aber ohne chirurgischen Eingriff im kompakten, ungefüllten Zustand durch den Mund in den Magen des Patienten eingeführt. Nachdem der Ballon in den Magen des Patienten eingeführt wurde, kann er durch einen kleinen, am Ballon angebrachten Füllschlauch mit einer sterilen Kochsalzlösung gefüllt werden. Dieser Schlauch wird nach dem Füllen entfernt. Die Präsenz eines aufgeblasenen Ballons im Magen gibt dem Patienten ein ständiges Sättigungsgefühl, so dass die Nahrungsaufnahme weniger häufig erfolgt. Jedoch hat auch dieses Verfahren erhebliche Nachteile, denn der ständige Kontakt des Ballons aus Kunststoff mit den inneren Wänden des Magens kann zu Magengeschwüren, Darmverschlüssen oder Magenschleimhautentzündungen führen. Auch kann der Ballon im Magen des Patienten platzen, was zumindest mit bedeutenden Unannehmlichkeiten für den Patienten verbunden ist.

Das laparoskopische Magenbanding ist ein weiteres Verfahren zur Bekämpfung der Fettleibigkeit, bei welchem im oberen Teil des Magens ein flexibles Band aus weichem Kunststoff (meistens Silikon) um den Magen des Patienten angebracht wird, so dass der Magen vom Band vollständig umschlungen wird. Das Magenband beinhaltet eine Kammer, welche sich durch die Zugabe der Flüssigkeit ausdehnen kann, womit der Innendurchmesser des Magenbandes verengt wird. Andererseits kann der Innendurchmesser des Magenbandes durch die Entnahme der Flüssigkeit aus der dehnbaren Kammer auch vergrössert werden. Zu diesem Zweck verbindet ein Schlauch das flexible Magenband mit einem Flüssigkeitsbehälter (auch Portkammer oder Portreservoir genannt), welcher unterhalb der Haut an einer leicht zugänglichen Stelle angebracht wird. Vorteil des laparoskopischen Magenbandings ist es, dass der Patient schon nach einer geringen Menge an Nahrung gesättigt ist und nicht weiter essen kann. Ausserdem wird durch die vorherrschende laparoskopische Implantation des Magenbandes im Vergleich zu klassischen chirurgischen Verfahren eine wesentlich kleinere Traumatisierung des Patienten bewirkt. Jedoch weist auch dieses Verfahren einen entscheidenden Nachteil auf, nämlich, dass die Magenöffnung des Patienten nicht präzise gesteuert bzw. kontrolliert werden kann. Darüber hinaus muss zur Anpassung der Magenöffnung des Patienten ein externer Eingriff zur Flüssigkeitszugabe bzw. -entnahme (meist durch eine Nadel) ausgeführt werden. Aus diesem Grund muss die Anpassung der Magenöffnung des Patienten jeweils ausschliesslich von spezialisierten Fachkräften (Ärzten) durchgeführt werden. Es ist daher klar, dass die Anpassung der Magenöffnung einerseits nicht zu beliebiger Zeit und andererseits nicht an beliebigem Ort durchgeführt werden kann. Auch kann das Magenband nicht an vorübergehende Bedürfnisse des Patienten angepasst werden.

Es sind daher bereits Einrichtungen vorgestellt worden, welche eine Regulierung der Flüssigkeitsmenge in einem Magenband ohne einen externen Eingriff ermöglichen. Eine solche Einrichtung gemäß dem Oberbegriff des Anspruchs 1 wird beispielsweise im Dokument WO 00/09049 beschrieben. Diese Einrichtung umfasst neben dem Magenband mit einer dehnbaren Kammer auch einen Behälter und ein hydraulisches Mittel, mittels welches die Flüssigkeit aus dem Behälter in das Magenband und umgekehrt verschoben werden kann. Insbesondere kann das hydraulische Mittel derart ausgebildet sein, dass die Wände des Behälter zur Änderung des Volumens verschoben werden können. Allerdings muss das hydraulische Mittel in dieser Einrichtung manuell bzw. über eine Fernbedienung gesteuert werden. Eine Er fassung der Körperlage des Patienten oder des Druckes auf die innere Wand der Speiseröhre des Patienten und die dynamische Anpassung der Magenöffnung aufgrund dieser Grössen ist also, nicht möglich.

### Offenbarung der Erfindung

Es ist eine Aufgabe der vorliegenden Erfindung, eine neue Einrichtung zur automatisierten Anpassung der Magenöffnung eines Patienten vorzuschlagen, welche nicht die Nachteile des Standes der Technik aufweisen. Es ist insbesondere eine Aufgabe der vorliegenden Erfindung, eine adaptive Einrichtung bereitzustellen, welche eine genaue, flexible und einfache vollständig automatisierte Änderung der Magenöffnung des Patienten ohne Bedarf an externen Eingriffen ermöglichen, wobei der Patient diese Änderung nicht willentlich beeinflussen kann.

Gemäss der vorliegenden Erfindung werden diese Ziele insbesondere durch die Elemente der unabhängigen Ansprüche erreicht. Weitere vorteilhafte Ausführungsvarianten gehen ausserdem aus den abhängigen Ansprüchen und der Beschreibung hervor.

Insbesondere werden diese Ziele durch die Erfindung dadurch erreicht, dass in einer adaptiven Einrichtung zur automatisierten Anpassung der Magenöffnung eines Patienten, umfassend ein Magenband mit einem nicht dehnbaren Rückenteil und einer ersten dehnbaren Kammer, und eine mit der ersten dehnbaren Kammer verbundene zweite dehnbare Kammer, wobei das Magenband zur Anpassung der Magenöffnung des Patienten um den Magen des Patienten herum platzierbar ist, und die Flüssigkeit mittels einer Beförderungsvorrichtung aus der einen dehnbaren Kammer in die andere dehnbare Kammer verschiebbar ist, wobei zur Aktivierung der Beförderungsvorrichtung eine Schaltvorrichtung mit einem Sensormodul vorgesehen ist, wobei mittels des Sensormoduls eine Messgrösse registrierbar und die Schaltvorrichtung basierend auf der Änderung der Messgrösse automatisiert steuerbar ist, wobei zur Aktivierung der Beförderungsvorrichtung eine Schaltvorrichtung mit einem Sensormodul vorgesehen ist, wobei mittels des Sensormoduls eine Messgrösse registrierbar und die Schaltvorrichtung basierend auf der Änderung der Messgrösse automatisiert steuerbar ist, mittels des Sensormoduls die Änderung der Körperlage des Patienten oder die Änderung des Druckes auf die innere Wand der Speiseröhre des Patienten registrierbar ist.

Der Vorteil einer solchen adaptiven Einrichtung liegt insbesondere darin, dass die Anpassung der Magenöffnung eines Patienten vollkommen automatisiert ausgeführt werden kann. Dadurch kann diese Anpassung auch ohne irgendwelche äussere Eingriffe ablaufen. Auf der einen Seite wird bei einer solchen adaptiven Einrichtung die Gesamtmenge an Flüssigkeit im geschlossenen System nicht verändert, was bei den herkömmlichen Einrichtungen nicht der Fall ist. Auf der anderen Seite kann eine beliebige Art von Steuerung eingebaut werden, so dass die Lebensqualität der Patienten wesentlich verbessert werden kann, ohne dass die erwünschten Effekte im Bezug auf eine effiziente Bekämpfung der krankhaften Fettleibigkeit vermindert eliminiert werden. Ausserdem kann die automatisierte Anpassung der Magenöffnung des Patienten in einer solchen adaptiven Einrichtung lediglich durch die Körperlageänderung des Patienten und/oder die Änderung des Druckes auf die innere Wand der Speiseröhre des Patienten (aufgrund der sich darin befindenden Nahrung) gesteuert werden. Insbesondere erlaubt diese Ausführungsvariante eine Anpassung der Magenöffnung des Patienten in Abhängigkeit davon, ob der Patient aufrecht steht bzw. sitzt oder liegt. So kann die Magenöffnung des Patienten beispielsweise bei einem Wechsel aus der sitzenden in die liegende Lage etwas vergrössert werden. Dadurch können die sich immer noch in der Speiseröhre befindenden Nahrungsreste etwas einfacher in den Magen befördert werden. Dies ist insbesondere abends und nachts vorteilhaft, da der Patient dadurch einen viel besseren Schlaf erreichen kann. Anders kann die Magenöffnung des Patienten bei der Änderung aus der Liegelage in die Sitz- oder Stehlage (z.B. morgens beim Aufstehen aus dem Bett) wieder verkleinert werden, womit sich die Nahrungsaufnahme erneut etwas schwieriger gestaltet. Andererseits kann bei einem übermässigen Druck auf die Innenwand der Speiseröhre des Patienten dessen Magenöffnung schnell etwas geöffnet werden, wodurch der Nahrungsstau schneller abgebaut werden kann. Die Magenöffnung kann anschliessend ebenso schnell und unkompliziert wieder etwas geschmälert werden, sobald der Druck auf die innere Wand der Speiseröhre des Patienten unter einen vorbestimmten Wert sinkt. Viele bekannte Schwierigkeiten und Probleme mit der Nahrungsstauung in der Speiseröhre und dem oberen Magenabschnitt bei den herkömmlichen Einrichtungen können bei dieser Ausführungsvariante auf eine besonders vorteilhafte Art und Weise beseitigt werden.

In einer weiteren Ausführungsvariante ist die Beförderungsvorrichtung mechanisch und/oder elektrisch angetrieben. Diese Ausführungsvariante hat unter anderem den Vorteil, dass mit verschiedenen Antriebstypen eine optimale und an die individuellen Bedürfnisse jedes Patienten angepasste Lösung angestrebt werden kann. Der mechanische Antrieb hat insbesondere den Vorteil, dass die Abhängigkeit von der Stromversorgung vermieden werden kann. Mittels eines kombinierten mechanisch-elektrischen Antriebs kann auf der anderen Seite die adaptive Einrichtung besonders einfach betrieben werden. Ein rein elektrischer Antrieb ist grundsätzlich weniger anfällig auf mögliche Störungen oder Defekte, wodurch eine höhere Zuverlässigkeit der adaptiven Einrichtung zur Anpassung der Magenöffnung des Patienten erreicht werden kann.

In einer anderen Ausführungsvariante ist die Beförderungsvorrichtung als eine hydraulische Pumpe ausgebildet. Diese Ausführungsvariante hat unter anderem den Vorteil, dass die Flüssigkeit zwischen der einen dehnbaren Kammer und der anderen dehnbaren Kammer auf eine besonders vorteilhafte und sichere Art und Weise verschoben werden kann. Es existieren heute auch miniaturisierte hydraulische Pumpen, so dass die Gesamtgrösse bzw. -masse der adaptiven Einrichtung im Vergleich zu den herkömmlichen Einrichtungen nicht übermässig vergrössert wird.

In einer anderen Ausführungsvariante umfasst die adaptive Einrichtung eine Stromspeicherungsvorrichtung zum Antrieb der Beförderungsvorrichtung, wobei die Beförderungsvorrichtung mit der Stromspeicherungsvorrichtung elektrisch verbunden ist. Diese Ausführungsvariante hat insbesondere den Vorteil, dass die Stromversorgung für die Beförderungsvorrichtung lokal erfolgen kann. Es werden daher keine externen Stromquellen benötigt, welche bei einem Ausfall die gesamte Einrichtung unbrauchbar machen würden. Diese Ausführungsvariante kann daher besonders vorteilhaft zur Anpassung der Magenöffnung des Patienten eingesetzt werden.

In einer wieder anderen Ausführungsvariante ist die Stromspeicherungsvorrichtung wiederaufladbar. Diese Ausführungsvariante hat unter anderem den Vorteil, dass die Stromspeicherungsvorrichtung nicht nach jeder vollständigen Entleerung ausgewechselt werden muss. Die Wiederaufladung der Stromspeicherungsvorrichtung kann unter Umständen auch auf kontaktlosem Wege realisiert werden. Durch diese Ausführungsvariante wird daher nicht nur die Handhabung der adaptiven Einrichtung zur Anpassung der Magenöffnung des Patienten wesentlich vereinfacht, sondern auch dessen Betriebskosten um ein Vielfaches reduziert.

In einer wieder weiteren Ausführungsvariante umfasst die adaptive Einrichtung eine Leitung zum Verbinden der ersten dehnbaren Kammer mit der zweiten dehnbaren Kammer. Diese Ausführungsvariante hat insbesondere den Vorteil, dass die beiden dehnbaren Kammern nicht zwingend dicht aneinander angebracht werden müssen. Insbesondere ist es möglich, dass die eine der beiden dehnbaren Kammern nicht als ein Bestandteil des Magenbandes, sondern als eine gesonderte Einheit ausgeführt wird. Mittels einer Leitung können dann die beiden Kammern miteinander verbunden werden, womit die notwendige Kommunikation hergestellt werden kann. Durch diese Ausführungsvariante können herkömmliche Einrichtungen zur Anpassung der Magenöffnung des Patienten mit relativ geringen Änderungen zu erfindungsgemässen adaptiven Einrichtungen zur Anpassung der Magenöffnung des Patienten umgebaut werden, womit eine weitere Kosteneinsparung bewirkt werden kann.

In einer anderen Ausführungsvariante umfasst die Leitung ein Ventil, wobei das Ventil durch die Schaltvorrichtung steuerbar ist. Diese Ausführungsvariante hat insbesondere den Vorteil, dass die Verschiebung der Flüssigkeit aus der einen dehnbaren Kammer in die andere dehnbare Kammer nicht ohne eine explizite Steuerung geschehen kann. Dadurch wird erreicht, dass die Magenöffnung nicht unwillentlich angepasst wird, was Komplikationen beim Patienten verursachen würde. Das Ventil kann insbesondere auch als ein Feinstellventil ausgeführt werden, womit die Menge an Flüssigkeit, die zwischen den beiden dehnbaren Kammern verschoben wird, besonders präzise kontrolliert werden kann. Auch kann durch eine permanente Deaktivierung des Ventils eine Verschiebung der Flüssigkeit zwischen den beiden dehnbaren Kammern vollständig verunmöglicht werden, womit die adaptive Einrichtung gemäss der vorliegenden Ausführungsvariante auch als eine herkömmliche Einrichtung verwendet werden kann.

In einer wieder anderen Ausführungsvariante ist ein Verzögerungsmodul vorgesehen, mittels welchem die Aktivierung der Schaltvorrichtung kontrollierbar ist. Der Vorteil dieser Ausführungsvariante liegt insbesondere darin, dass die automatisierte Anpassung der Magenöffnung des Patienten nicht einfach durch den Patienten ausgelöst werden kann, indem sich dieser während einer kurzen Zeit willentlich in eine liegende Position begibt. Das erfindungsgemässe Verzögerungsmodul sorgt dafür, dass die automatisierte Änderung der Magenöffnung des Patienten erst nach einer bestimmten Zeit einsetzt, so dass die therapeutischen Vorteile der adaptiven Einrichtung keinesfalls beeinträchtigt werden. Als Verzögerungsmodul eignen sich alle Vorrichtungen oder Geräte, welche in den Körper des Patienten implantiert werden können.

In einer weiteren Ausführungsvariante ist das Verzögerungsmodul programmierbar. Der Vorteil dieser Ausführungsvariante liegt unter anderem darin, dass die Verzögerung bei jedem Patienten nach Bedarf anders eingestellt werden kann. Selbstverständlich ist es bei gewissen Patienten notwendig, die Verzögerung relativ gross zu wählen, da sie Schlafschwierigkeiten haben, so dass eine vorzeitige Öffnung nicht im Interesse der Behandlung wäre. Andererseits ist es bei gewissen Patienten notwendig, die Verzögerung möglichst klein zu machen, da sie auf eine schnelle Öffnung bzw. Schliessung der Magenöffnung angewiesen sind. Ein solch programmierbares Verzögerungsmodul erlaubt also eine sehr einfache und zuverlässige Individualisierung und Personalisierung der adaptiven Einrichtung zur automatisierten Anpassung der Magenöffnung des Patienten.

In einer wieder weiteren Ausführungsvariante ist das Verzögerungsmodul mittels einer Fernbedienung programmierbar und/oder steuerbar. Diese Ausführungsvariante hat unter anderem den Vorteil, dass die Programmierung des Verzögerungsmoduls (und damit auch die Länge der Verzögerung bei der automatisierten Anpassung der Magenöffnung des Patienten) auch extern vorgenommen werden kann, ohne dass dafür ein Eingriff in den Körper des Patienten notwendig wäre. Insbesondere kann dadurch einer kontinuierlichen Verbesserung beim Patienten Rechnung getragen werden.

In einer wieder anderen Ausführungsvariante ist die adaptive Einrichtung im Wesentlichen aus Kunststoff gebildet. Diese Ausführungsvariante hat unter anderem den Vorteil, dass der Kunststoff besonders vorteilhafte Eigenschaften besitzt, welche insbesondere bei der Implantation und den anschliessenden Betrieb der adaptiven Einrichtung zur Anpassung der Magenöffnung des Patienten zum Ausdruck kommen. Besonders geeignete Kunststoffe sind Silikon und Silikonelastomere, welche bereits bei vielen anderen implantierbaren Einrichtungen erfolgreich eingesetzt werden.

### Kurze Beschreibung der Zeichnungen

Nachfolgend werden die Ausführungsvarianten der vorliegenden Erfindung anhand von Beispielen beschrieben. Die Beispiele der Ausführungen werden durch folgende beigelegte Figuren illustriert:
Figur 1 zeigt eine perspektivische schematische Darstellung einer Einrichtung zur Anpassung der Magenöffnung eines Patienten aus dem Stand der Technik.
Figur 2 zeigt eine Draufsicht eines Magenbandes einer Einrichtung zur Anpassung der Magenöffnung eines Patienten aus dem Stand der Technik im geschlossenen Zustand.
Figur 3 zeigt eine perspektivische schematische Darstellung des Magens eines menschlichen Körpers mit der implantierten erfindungsgemässen Einrichtung zur Anpassung der Magenöffnung eines Patienten.
Figur 4 zeigt eine perspektivische schematische Darstellung einer Einrichtung zur automatisierten Anpassung der Magenöffnung eines Patienten gemäss einer Ausführungsvariante der vorliegenden Erfindung.
Figur 5 zeigt eine perspektivische schematische Darstellung einer Einrichtung zur automatisierten Anpassung der Magenöffnung eines Patienten gemäss einer weiteren Ausführungsvariante der vorliegenden Erfindung.
Figur 6 zeigt die Seitenansicht eines Teils der Einrichtung zur automatisierten Anpassung der Magenöffnung eines Patienten aus Figur 5:
Figur 6A zeigt einen Teil der Einrichtung zur automatisierten Anpassung der Magenöffnung des Patienten bei der Vergrösserung der Magenöffnung.
Figur 6B zeigt einen Teil der Einrichtung zur automatisierten Anpassung der Magenöffnung des Patienten bei der Verkleinerung der Magenöffnung.

### Ausführungsvarianten der Erfindung

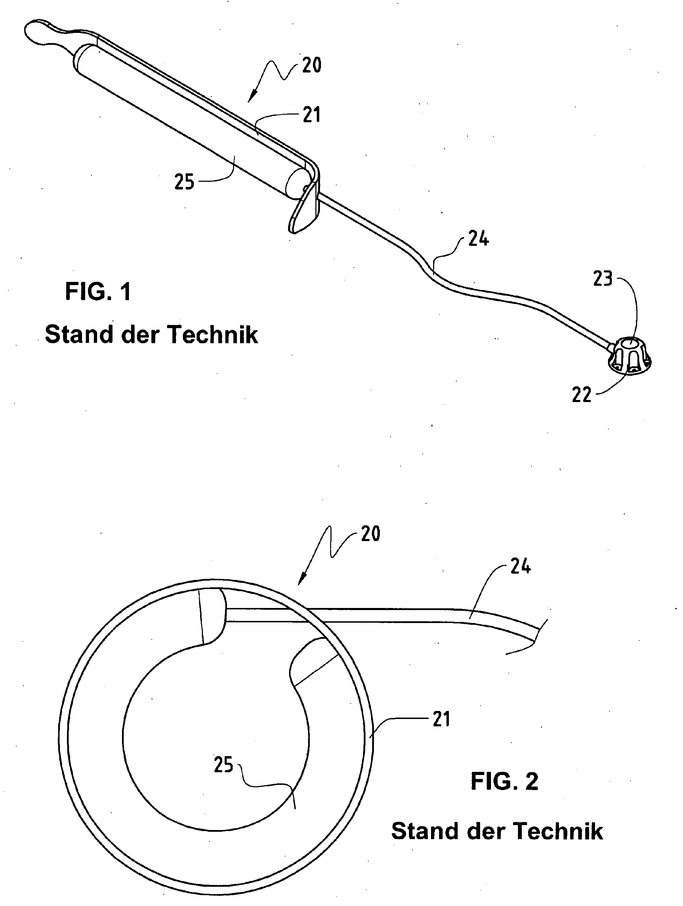

In Figur 1 ist eine Einrichtung zur Anpassung der Magenöffnung eines Patienten aus dem Stand der Technik dargestellt. In Figur 1 bezieht sich das Bezugszeichen 20 auf das Magenband, das Bezugszeichen 21 auf einen nicht dehnbaren Rückenteil an der Aussenseite des Magenbands 20 und das Bezugszeichen 25 an eine erste dehnbare Kammer an der Innenseite des Magenbands 20. Des Weiteren bezieht sich das Bezugszeichen 22 auf die so genannte Portkammer bzw. das Portreservoir zur Flüssigkeitsaufnahme und das Bezugszeichen 23 auf die entsprechende Membran, welche mittels einer Nadel zur Flüssigkeitszufuhr oder -entnahme perkutan durchstochen werden kann. Darüber hinaus bezieht sich das Bezugszeichen 24 auf eine Leitung, beispielsweise einen dünnen Schlauch, welcher die Portkammer 22 mit dem Magenband 20 bzw. mit der ersten dehnbaren Kammer 25 verbindet.

Figur 2 illustriert einen vergrösserten Ausschnitt der Einrichtung zur Anpassung der Magenöffnung eines Patienten, wobei in Figur 2 das Magenband 20 im geschlossenen Zustand dargestellt wird. Im geschlossenen Zustand bildet das Magenband 20 eine Schlinge, wobei der nicht dehnbare Rückenteil 21 des Magenbandes 20 an der Aussenseite der Schlinge, und die erste dehnbare Kammer 25 an der Innenseite der Schlinge zu liegen kommt. Durch den dünnen Schlauch 24 ist die erste dehnbare Kammer 25 mit der Portkammer 22 verbunden.

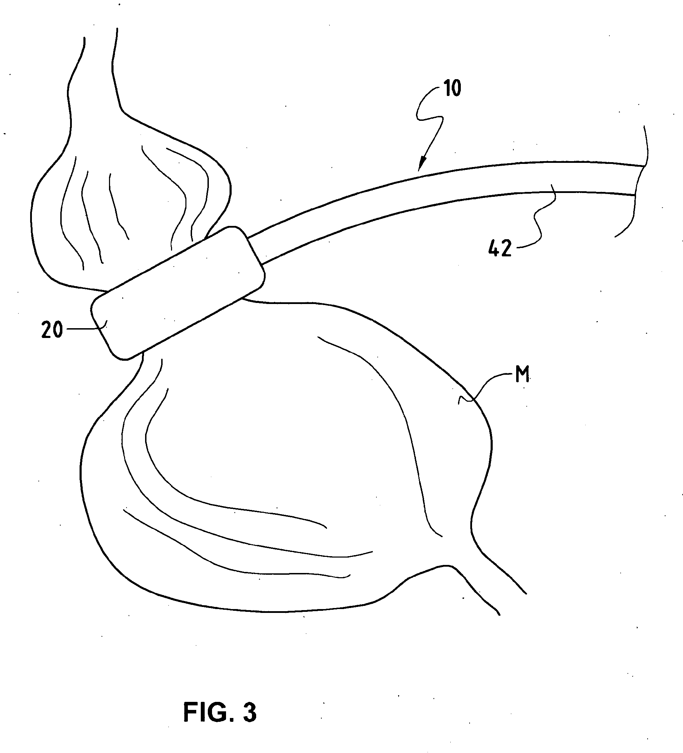

Das Magenband 20 wird in einer laparoskopischen Operation um den oberen Teil des Magens (M) des Patienten gelegt, wodurch der Magen (M) in zwei Hälften unterteilt wird, wie in Figur 3 vereinfacht dargestellt wird. Dabei wird der wesentlich kleinere obere Anteil (Vormagen) durch die künstlich geschaffene Enge des Magenbandes 20 von dem unteren Anteil (Restmagen) getrennt. Über den Schlauch 24 wird das Magenband 20, bzw. die erste dehnbare Kammer 25 mit der Portkammer 22 verbunden, wobei die Portkammer 22 unter die Haut eingepflanzt wird. Insbesondere eignet sich die Bauchregion des Patienten für die Einpflanzung der Portkammer 22, da die fettleibigen Patienten unterhalb der Haut meistens über eine dicke Fettschicht verfügen. Über die Verbindung zwischen der Portkammer 22 und der ersten dehnbaren Kammer 25 des Magenbandes 20 kann die Füllung des Magenbandes 20 (bzw. der ersten dehnbaren Kammer 25) gesteuert werden, d.h. den individuellen Bedürfnissen eines jeden Patienten angepasst werden. Zur Füllung des Magenbandes 20 wird die Portkammer 22 bzw. die Membran 23 mit einer feinen Nadel (zum Teil unter Röntgenkontrolle) durch die Haut punktiert und Flüssigkeit hinzugefügt oder abgezogen, womit die erste dehnbare Kammer 25 entweder gefüllt oder entleert wird. Auf diese Weise kann die Grösse der Öffnung des Magenbandes 20, d.h. sein Innendurchmesser verändert werden, wodurch auch die Magenöffnung (auch Stoma genannt) angepasst werden kann. Das Magenband 20 besteht üblicherweise aus weichem Kunststoff, meistens aus Silikon, wobei auch andere Materialien denkbar wären.

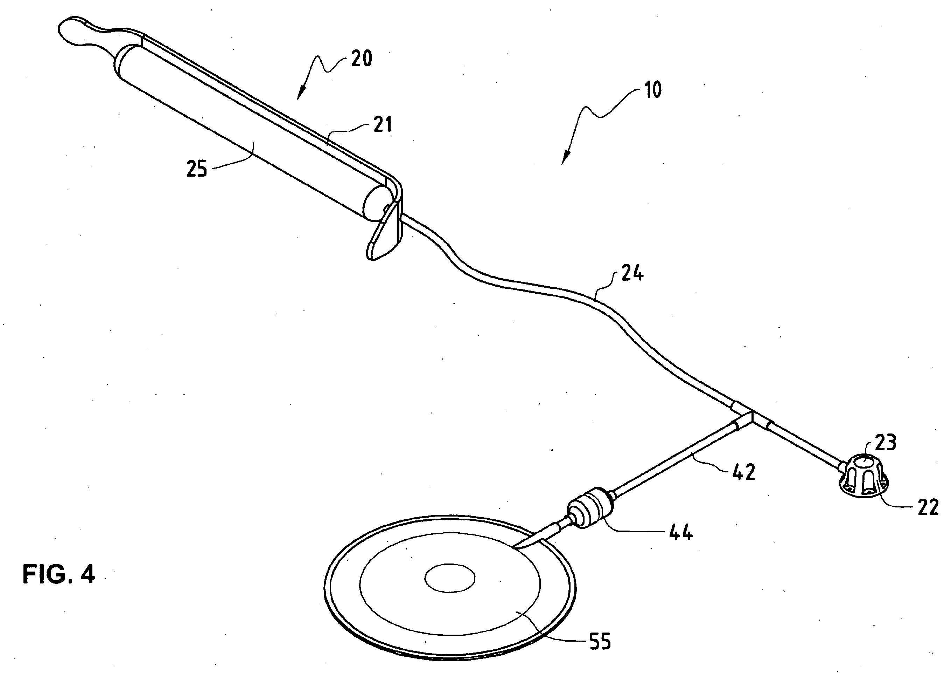

Figur 4 illustriert eine Einrichtung zur automatisierten Anpassung der Magenöffnung eines Patienten gemäss einer Ausführungsvariante der vorliegenden Erfindung. In Figur 4 bezieht sich das Bezugszeichen 20 auf das Magenband, das Bezugszeichen 21 auf einen nicht dehnbaren Rückenteil an der Aussenseite des Magenbands 20 und das Bezugszeichen 25 an eine erste dehnbare Kammer an der Innenseite des Magenbands 20. Ferner bezieht sich das Bezugszeichen 22 auf die so genannte Portkammer bzw. das Portreservoir zur Flüssigkeitsaufnahme und das Bezugszeichen 23 auf die entsprechende Membran, welche mittels einer Nadel zur Flüssigkeitszufuhr oder -entnahme perkutan durchstochen werden kann Des Weiteren bezieht sich das Bezugszeichen 24 auf eine Leitung, beispielsweise einen dünnen Schlauch, welcher die Portkammer 22 mit dem Magenband 20 bzw. mit der ersten dehnbaren Kammer 25 verbindet. Ausserdem bezieht sich in Figur 4 das Bezugszeichen 55 auf eine zweite dehnbare Kammer, das Bezugszeichen 42 auf eine Leitung, beispielsweise einen dünnen Schlauch, über welchen eine Verbindung zwischen der zweiten dehnbaren Kammer 55 und der ersten dehnbaren Kammer 25 hergestellt werden kann und das Bezugszeichen 44 auf ein Ventil.

Zur Anpassung der Magenöffnung des Patienten kann die Flüssigkeit aus der ersten dehnbaren Kammer 25 in die zweite dehnbare Kammer 55 und umgekehrt über die Leitungen 24 respektive 42 verschoben werden. Wenn die Flüssigkeit beispielsweise aus der ersten dehnbaren Kammer 25 in die zweite dehnbare Kammer 55 verschoben wird, so verkleinert sich die Menge an Flüssigkeit in der ersten dehnbaren Kammer 25, was wiederum eine Vergrösserung des Innendurchmessers des Magenbandes 20 zur Auswirkung hat. Durch diese Vergrösserung des Innendurchmessers des Magenbandes 20 wird der Druck auf den Magen (M) des Patienten etwas reduziert, womit die Magenöffnung des Patienten etwas vergrössert wird. Dadurch kann auch die Aufnahme der Nahrung in den Magen (M) des Patienten etwas vereinfacht und beschleunigt werden. Andererseits, wenn die Flüssigkeit umgekehrt aus der zweiten dehnbaren Kammer 55 in die erste dehnbare Kammer 25 verschoben wird, so vergrössert sich die Menge der Flüssigkeit in der ersten dehnbaren Kammer 25, was wiederum eine Verkleinerung des Innendurchmessers des Magenbandes 20 zur Auswirkung hat. Durch diese Verkleinerung des Innendurchmessers des Magenbandes 20 nimmt auch der Druck auf den Magen (M) des Patienten etwas zu, womit die Magenöffnung des Patienten etwas verkleinert wird. Dadurch gestaltet sich die Aufnahme der Nahrung in den Magen (M) des Patienten wieder etwas schwieriger und langsamer.

Das Ventil 44 befindet sich beispielsweise an der Leitung 42, mittels welcher die zweite dehnbare Kammer 55 mit der ersten dehnbaren Kammer 25 verbunden ist. Selbstverständlich kann sich aber das Ventil 44 auch an einem anderen Ort befinden, solange seine Funktionalität damit nicht eingeschränkt wird. Das Ventil 44 kann nach Bedarf geöffnet oder geschlossen werden. Durch eine Öffnung des Ventils 44 kann eine Verschiebung der Flüssigkeit aus der ersten dehnbaren Kammer 25 in die zweite dehnbare Kammer überhaupt erst ermöglicht werden. Andererseits kann durch eine permanente Schliessung des Ventils 44 eine Verschiebung der Flüssigkeit zwischen den beiden dehnbaren Kammern 25, 55 blockiert werden, womit eine erfindungsgemässe Einrichtung 10 zur Anpassung der Magenöffnung des Patienten als eine herkömmliche Einrichtung benutzt werden kann.

Die zweite dehnbare Kammer 55, wie allerdings auch die erste dehnbare Kammer 25, kann aus einem hochelastischen Material gebaut werden, so dass sie eine intrinsische Spannung aufweist. Diese intrinsische Spannung der beiden dehnbaren Kammern 25, 55 kann zur Verschiebung der Flüssigkeit aus der einen dehnbaren Kammer 25, 55 in die zweite dehnbare Kammer 55, 25 verwendet werden. Selbstverständlich sollten aber in diesem Fall die beiden dehnbaren Kammern 25, 55 eine entgegengesetzte Spannung aufweisen (die erste Kammer 25 ist gespannt wenn die zweite dehnbare Kammer 55 entspannt ist und umgekehrt).

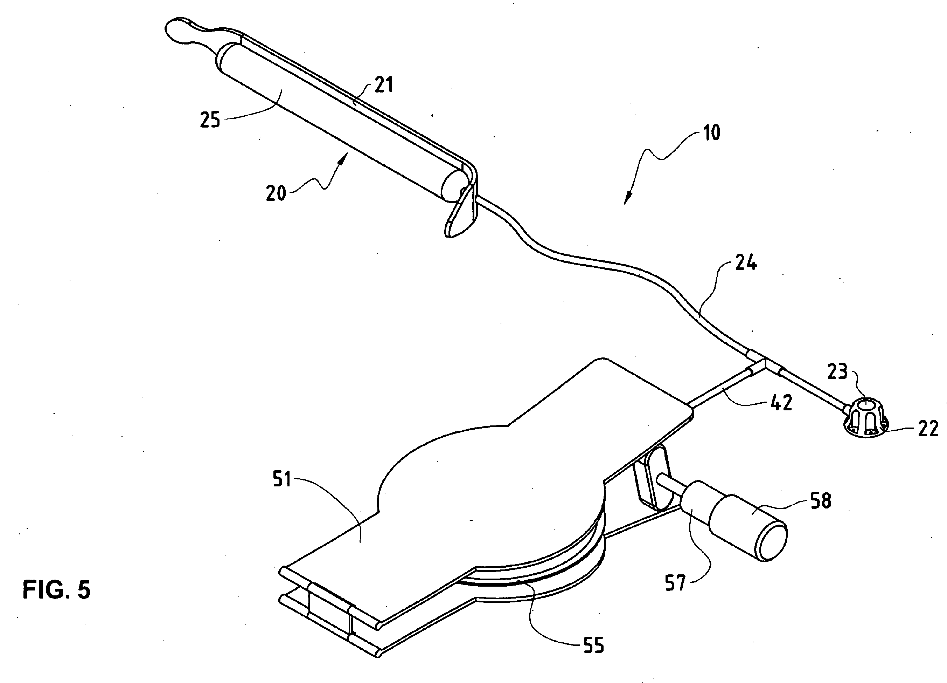

Figur 5 illustriert eine Einrichtung zur automatisierten Anpassung der Magenöffnung eines Patienten gemäss einer anderen Ausführungsvariante der vorliegenden Erfindung. In Figur 5 bezieht sich das Bezugszeichen 20 wieder auf das Magenband, das Bezugszeichen 21 auf einen nicht dehnbaren Rückenteil an der Aussenseite des Magenbands 20 und das Bezugszeichen 25 an eine erste dehnbare Kammer an der Innenseite des Magenbands 20. Darüber hinaus bezieht sich das Bezugszeichen 22 wieder auf die so genannte Portkammer bzw. das Portreservoir zur Flüssigkeitsaufnahme und das Bezugszeichen 23 auf die entsprechende Membran, welche mittels einer Nadel jeweils zur Flüssigkeitszufuhr oder -entnahme perkutan durchstochen werden kann. Ausserdem bezieht sich das Bezugszeichen 24 wieder auf eine Leitung, beispielsweise einen dünnen Schlauch, welcher die Portkammer 22 mit dem Magenband 20 bzw. mit der ersten dehnbaren Kammer 25 verbindet. Das Bezugszeichen 55 bezieht sich wieder auf eine zweite dehnbare Kammer und das Bezugszeichen 42 auf eine Leitung, beispielsweise einen dünnen Schlauch, über welchen eine Verbindung zwischen der zweiten dehnbaren Kammer 55 und der ersten dehnbaren Kammer 25 hergestellt werden kann. In Figur 5 bezieht sich das Bezugszeichen 51 auf eine Beförderungsvorrichtung, welche zur aktiven Verschiebung der Flüssigkeit aus der einen dehnbaren Kammer 25, 55 in die andere dehnbare Kammer 55, 25 eingesetzt werden kann, das Bezugszeichen 57 auf eine Schaltvorrichtung zur Aktivierung der Beförderungsvorrichtung 51, und das Bezugszeichen 58 auf eine Stromspeicherungsvorrichtung, welche beispielsweise zum Antrieb der Beförderungsvorrichtung 51 verwendet werden kann.

Wie bereits in der Ausführungsvariante aus Figur 4, kann zur Anpassung der Magenöffnung des Patienten die Flüssigkeit aus der ersten dehnbaren Kammer 25 in die zweite dehnbare Kammer 55 und umgekehrt über die Leitungen 24 respektive 42 verschoben werden. Mittels der Beförderungsvorrichtung 51 kann nun diese Verschiebung der Flüssigkeit zwischen den beiden dehnbaren Kammern 25, 55 auch aktiv angetrieben werden. Im dargestellten Beispiel befördert die Beförderungsvorrichtung 51 die Flüssigkeit aus der zweiten dehnbaren Kammer 55 zur ersten dehnbaren Kammer 55 auf eine mechanische Art und Weise. Die Beförderungsvorrichtung 51 besteht aus zwei Blättern aus einem harten Material (beispielsweise Stahl), welche so gespannt sind, dass sie im normalen Zustand (d.h. ohne Einwirkung von äusseren Einflüssen) gegeneinander gedrückt werden. Die zweite dehnbare Kammer 55 befindet sich zwischen den beiden Blättern der Beförderungsvorrichtung 51 und steht im normalen Zustand der Beförderungsvorrichtung 51 unter Druck. Im normalen Zustand der Beförderungsvorrichtung 51 wird also die Flüssigkeit aus der zweiten dehnbaren Kammer 55 in die erste dehnbare Kammer 25 verschoben. Allenfalls kann diese Verschiebung durch das Ventil 44, das sich an der Leitung 42 zwischen der zweiten dehnbaren Kammer 55 und der ersten dehnbaren Kammer 55 befindet, nach Bedarf blockiert werden.

In Figur 5 bezieht sich das Bezugszeichen 58 auf eine Stromspeicherungsvorrichtung. Die Stromspeicherungsvorrichtung 58 kann die elektrische Energie speichern, welche zum Antrieb der Beförderungsvorrichtung 51 nötig ist. Zu diesem Zweck ist die Stromspeicherungsvorrichtung 58 mit der Beförderungsvorrichtung 51 und/oder mit der Schaltvorrichtung 57 elektrisch verbunden. Diese Stromspeicherungsvorrichtung 58 kann wiederaufladbar sein, oder nach jeder Entleerung ausgewechselt werden. Insbesondere kann die Stromspeicherungsvorrichtung 58 eine Lithium-Ion-Batterie sein.

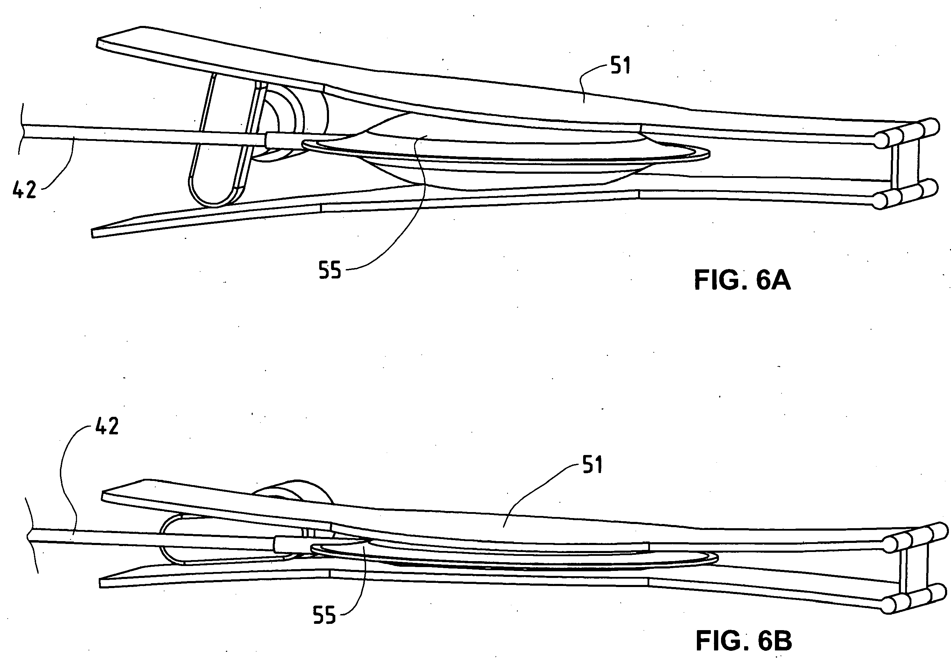

In Figur 5 bezieht sich das Bezugszeichen 57 auf eine Schaltvorrichtung mittels welcher die Beförderungsvorrichtung 51 aktiviert bzw. gesteuert und kontrolliert werden kann. Die Schaltvorrichtung 57 ist in Figur 5 mit einer Nockenscheibe verbunden, welche auch zwischen den beiden Blättern der Beförderungsvorrichtung 51 liegt. Mittels der Schaltvorrichtung 57 wird die Nockenscheibe in Bewegung gesetzt, wodurch auf die Blätter der Beförderungsvorrichtung 51 ein Druck ausgeübt wird und die Blätter auseinander gedrückt werden. Dadurch wird auch der Druck auf die ebenfalls zwischen den beiden Blättern der Beförderungsvorrichtung 51 liegende zweite dehnbare Kammer 55 verkleinert, so dass die Flüssigkeit aus der ersten dehnbaren Kammer 25 in die zweite dehnbare Kammer 55 zurück fliessen kann. Figur 6A zeigt die Beförderungsvorrichtung 51 in einer Seitenansicht, wenn die Blätter der Beförderungsvorrichtung 51 mittels der Nockenscheibe auseinander gedrückt sind, so dass sich die zweite dehnbare Kammer 55 aufgrund der intrinsischen Spannung ausdehnen kann. Andererseits zeigt Figur 6B die entgegengesetzte Situation, in welcher die zweite dehnbare Kammer 55 durch die beiden Blätter der Beförderungsvorrichtung 51 zusammen gepresst wird. Selbstverständlich kann auch diese Flüssigkeitsverschiebung mittels des Ventils 44 gegebenenfalls blockiert werden.

Jedoch möchten wir an dieser Stelle darauf hinweisen, dass sowohl die Beförderungsvorrichtung 51 als auch die Schaltvorrichtung 57 oder beliebige andere Elemente der erfindungsgemässen adaptiven Einrichtung zur Anpassung der Magenöffnung des Patienten selbstverständlich auch ganz andere Formen bzw. Funktionsweisen aufweisen können. So kann beispielsweise insbesondere die Beförderungsvorrichtung 51 auch eine hydraulische oder sonstige Pumpe oder eine weitere andersartige Vorrichtung sein, mittels welcher die Flüssigkeit zwischen den beiden dehnbaren Kammern 25, 55 hin und her verschoben werden kann.

Die Schaltvorrichtung 57 kann insbesondere basierend auf der Änderung einer Messgrösse automatisiert gesteuert werden. Dazu umfasst die Schaltvorrichtung 57 ein Sensormodul zur Registrierung dieser Messgrösse. Aufgrund des Werts dieser Messgrösse, bzw. aufgrund der Änderung dieser Messgrösse kann die Schaltvorrichtung 57 automatisiert gesteuert werden. Dieses Sensormodul kann aber selbstverständlich auch an einem anderen Ort platziert sein und mit der Schaltvorrichtung 57 drahtgebunden oder drahtlos verbunden sein. Die vom Sensormodul registrierte Messgrösse kann insbesondere die Körperlage des Patienten sein. In diesem Fall kann die Schaltvorrichtung 57 durch die Körperlage des Patienten so gesteuert sein, dass bei einer aufrechten (stehenden oder sitzenden) Körperposition das Magenband 20 einen kleinen Innendurchmesser aufweist, wodurch die Magenöffnung des Patienten klein gehalten wird. Diese Körperlage entspricht nämlich der normalen Tageslage des Patienten, bei welcher die Probleme relativ selten auftreten. Bei einer Körperlageänderung aus der aufrechten in eine waagerechte Position (liegend) kann die Schaltvorrichtung 57 so eingestellt sein, dass der Innendurchmesser des Magenbandes 20 etwas vergrössert wird, so dass die Magenöffnung des Patienten etwas vergrössert wird. Damit kann die Nahrungsaufnahme etwas vereinfacht werden, so dass keine Nahrungsstaus in der Speiseröhre des Patienten entstehen. Eine ähnliche Wirkung kann auch so erreicht werden, dass die Schaltvorrichtung 57 durch die Änderung des Drucks auf die Innenwand der Speiseröhre des Patienten gesteuert wird. Bei einem Druck, welcher über einem vordefinierten Niveau liegt kann die Magenöffnung des Patienten durch die Verschiebung der Flüssigkeit aus der ersten dehnbaren Kammer 25 in die zweite dehnbare Kammer 55 vergrössert werden, oder umgekehrt. Viele Probleme verbunden mit der Benutzung der Magenbänder 20, können dadurch eliminiert werden.

Auf der anderen Seite muss verhindert werden, dass der Patient die automatisierte Anpassung der Magenöffnung willentlich auslöst, um die Therapie beispielsweise auszutricksen. So könnte sich der Patient zum Beispiel kurz in die liegende Position begeben, und so die Öffnung der Magenöffnung bewirken Selbstverständlich würde die automatisierte Anpassung der Magenöffnung in diesem Fall nicht das ursprüngliche Ziel erfüllen. Deshalb kann in der erfindungsgemässen Einrichtung ein Verzögerungsmodul vorgesehen werden, mittels welchem die Aktivierung der Schaltvorrichtung kontrolliert werden kann. So kann die Anpassung der Magenöffnung des Patienten beispielsweise erst nach einer Startverzögerung von einer halben Stunde eingeleitet werden. Während dieser Verzögerungszeit würde die Aktivierung der Beförderungsvorrichtung 51 gehemmt, obwohl aufgrund der Signale des Sensormoduls der Schaltvorrichtung 57 eine entsprechende Aktivierung zu tätigen wäre. Selbstverständlich kann diese Verzögerung variabel gestaltet werden, beispielsweise indem die Verzögerungszeit bei der Erweiterung der Magenöffnung etwas grösser wäre, als die Verzögerungszeit bei der Schliessung der Magenöffnung des Patienten. Auch kann die Verzögerungszeit in einem oder im anderen Fall auch auf Null eingestellt werden, wodurch es zu gar keinen Verzögerung kommen würde. Diese Verzögerungszeit kann einmalig vor der Implantation bestimmt und programmiert werden, aber es können auch Verzögerungsmodule vorgesehen werden, welche beliebig programmiert und umprogrammiert werden können. Dabei kann diese Programmierung bzw. Steuerung des Verzögerungsmoduls auch mittels einer Fernbedienung durchgeführt werden. Diese Fernsteuerung kann beispielsweise mittels eines äusseren Senders und eines implantierbaren Empfängers realisiert werden, wobei der implantierbare Empfänger mit dem Verzögerungsmodul verbunden ist. Zur Signalübertragung können beispielsweise Radiowellen verwendet werden. Durch eine Fernsteuerung kann die Verzögerungszeit auch nach Bedarf (auch nur vorübergehend) geändert werden, ohne dass ein externer Eingriff notwendig werden.

Zum Schluss sei darauf hingewiesen, dass die beispielhaft beschriebenen Ausführungsvarianten nur eine Auswahl an möglichen Realisierungen der erfindungsgemässen Gedanken darstellen und keinesfalls als limitierend angeschaut werden sollen. Der Fachmann wird verstehen, dass viele andere Implementierungen der Erfindung möglich sind, ohne dass die wesentlichen Merkmale der Erfindung vernachlässigt werden.

## Patentansprüche

1. Adaptive Einrichtung (10) zur automatisierten Anpassung der Magenöffnung eines Patienten, umfassend ein Magenband (20) mit einem nicht dehnbaren Rückenteil (21) und einer ersten dehnbaren Kammer (25), und eine mit der ersten dehnbaren Kammer (25) verbundene zweite dehnbare Kammer (55), wobei das Magenband (20) zur Anpassung der Magenöffnung des Patienten um den Magen (M) des Patienten herum platzierbar ist, und die Flüssigkeit mittels einer Beförderungsvorrichtung (51) aus der einen dehnbaren Kammer (25, 55) in die andere dehnbare Kammer (55, 25) verschiebbar ist, **dadurch gekennzeichnet, dass** zur Aktivierung der Beförderungsvorrichtung (51) eine Schaltvorrichtung (57) mit einem Sensormodul vorgesehen ist, wobei mittels des Sensormoduls eine Messgrösse registrierbar und die Schaltvorrichtung (57) basierend auf der Änderung der Messgrösse automatisiert steuerbar ist, wobei
mittels des Sensormoduls die Änderung der Körperlage des Patienten oder die Änderung des Druckes auf die innere Wand der Speiseröhre des Patienten registrierbar ist.

2. Adaptive Einrichtung nach Anspruch 1, **dadurch gekennzeichnet, dass** die Beförderungsvorrichtung (51) mechanisch und/oder elektrisch angetrieben ist.

3. Adaptive Einrichtung nach Anspruch 1 oder 2, **dadurch gekennzeichnet, dass** die Beförderungsvorrichtung (51) als eine hydraulische Pumpe ausgebildet ist.

4. Adaptive Einrichtung nach einem der Ansprüche 1 bis 3, **dadurch gekennzeichnet, dass** die adaptive Einrichtung (10) eine Stromspeicherungsvorrichtung (58) zum Antrieb der Beförderungsvorrichtung (51) umfasst, wobei die Beförderungsvorrichtung (51) mit der Stromspeicherungsvorrichtung (58) elektrisch verbunden ist.

5. Adaptive Einrichtung nach Anspruch 4, **dadurch gekennzeichnet, dass** die Stromspeicherungsvorrichtung (58) wiederaufladbar ist.

6. Adaptive Einrichtung nach einem der Ansprüche 1 bis 5, **dadurch gekennzeichnet, dass** die adaptive Einrichtung (10) eine Leitung (42) zum Verbinden der ersten dehnbaren Kammer (25) mit der zweiten dehnbaren Kammer (55) umfasst.

7. Adaptive Einrichtung nach Anspruch 6, **dadurch gekennzeichnet, dass** die Leitung (42) ein Ventil (44) umfasst, wobei das Ventil (44) durch die Schaltvorrichtung (57) steuerbar ist.

8. Adaptive Einrichtung nach einem der Ansprüche 1 bis 7, **dadurch gekennzeichnet, dass** ein Verzögerungsmodul vorgesehen ist, mittels welchem die Aktivierung der Schaltvorrichtung (57) kontrollierbar ist.

9. Adaptive Einrichtung nach Anspruch 8, **dadurch gekennzeichnet, dass** das Verzögerungsmodul programmierbar ist.

10. Adaptive Einrichtung nach Anspruch 8 oder 9, **dadurch gekennzeichnet, dass** das Verzögerungsmodul mittels einer Fernbedienung programmierbar und/oder steuerbar ist.

11. Adaptive Einrichtung nach einem der Ansprüche 1 bis 10, **dadurch gekennzeichnet, dass** die adaptive Einrichtung (10) im Wesentlichen aus Kunststoff gebildet ist.

## Claims

1. Adaptive device (10) for automated adaptation of the stomach opening of a patient, comprising a gastric band (20) with a non-elastic back part (21) and a first expandable chamber (25), and a second expandable chamber (55) connected to the first expandable chamber (25), the gastric band (20) being placeable around the stomach (M) of the patient for adaptation of the stomach opening of the patient, and the fluid being displaceable from the one expandable chamber (25, 55) into the other expandable chamber (55, 25) by means of a conveyance device (51), **characterised in that** a switching device (57) with a sensor module is provided for activation of the conveyance device (51), a measurement value being able to be registered by means of the sensor module, and the switching device (57) being controllable in an automated way based on the change in the measurement value,
the change in the position of the body of the patient and/or the change in the pressure on the inner wall of the oesophagus of the patient being able to be registered by means of the sensor module.

2. Adaptive device according to claim 1, **characterised in that** the conveyance device (51) is driven mechanically and/or electrically.

3. Adaptive device according to claim 1 or 2, **characterised in that** the conveyance device (51) is designed as a hydraulic pump.

4. Adaptive device according to one of the claims 1 to 3, **characterised in that** the adaptive device (10) comprises a power storage device (58) for driving the conveyance device (51), the conveyance device (51) being electrically connected to the power storage device (58).

5. Adaptive device according to claim 4, **characterised in that** the power storage device (58) is rechargeable.

6. Adaptive device according to one of the claims 1 to 5, **characterised in that** the adaptive device (10) comprises a line (42) for connection of the first expandable chamber (25) to the second expandable chamber (55).

7. Adaptive device according to claim 6, **characterised in that** the line (42) comprises a valve (44), the valve (44) being controllable by the switching device (57).

8. Adaptive device according to one of the claims 1 to 7, **characterised in that** a delay module is provided by means of which the activation of the switching device (57) is controllable.

9. Adaptive device according to claim 8, **characterised in that** the delay module is programmable.

10. Adaptive device according to claim 8 or 9, **characterised in that** the delay module is programmable and/or controllable by means of a remote control.

11. Adaptive device according to one of the claims 1 to 10, **characterised in that** the adaptive device (10) is made substantially of synthetic material.

## Revendications

1. Dispositif adaptatif (10) pour l'adaptation automatique de l'ouverture de l'estomac d'un patient, comprenant un anneau gastrique (20) avec une partie arrière non dilatable (21) et une première cavité dilatable (25) et une seconde cavité dilatable (55) reliée à la première cavité dilatable (25), l'anneau gastrique (20) pouvant être placé autour de l'estomac (M) du patient et être adapté à l'ouverture gastrique du patient, le liquide pouvant être déplacé au moyen d'un dispositif de transport (51) depuis une cavité dilatable (25, 55) à l'autre cavité dilatable (55, 25), **caractérisé en ce que** pour l'activation du dispositif de transport (51), il est prévu un dispositif de commande (57) avec un module détecteur, au moyen du module détecteur une grandeur de mesure pouvant être enregistrée et le dispositif de commutation (57) pouvant être commandé de manière automatisée sur la base de la modification de la grandeur de mesure,
**caractérisé en ce qu'**au moyen du module de détection la modification de la position du corps du patient ou la modification de la pression sur la paroi interne du tube digestif du patient peuvent être enregistrées.

2. Dispositif adaptatif selon la revendication 1, **caractérisé en ce que** le dispositif de transport (51) est entraîné mécaniquement et/ou électriquement.

3. Dispositif adaptatif selon la revendication 1 ou 2, **caractérisé en ce que** le dispositif de transport (51) est réalisé comme une pompe hydraulique.

4. Dispositif adaptatif selon l'une des revendications 1 à 3, **caractérisé en ce que** le dispositif adaptatif (10) comprend un dispositif de stockage de courant (58) pour l'entraînement du dispositif de transport (51), le dispositif de transport (51) étant relié électriquement au dispositif de stockage de courant (58).

5. Dispositif adaptatif selon la revendication 4, **caractérisé en ce que** le dispositif de stockage de courant (58) est rechargeable.

6. Dispositif adaptatif selon l'une des revendications 1 à 5, **caractérisé en ce que** le dispositif adaptatif (10) comprend une conduite (42) pour relier la première cavité dilatable (25) à la seconde cavité dilatable (55).

7. Dispositif adaptatif selon la revendication 6, **caractérisé en ce que** la conduite (42) comprend une valve (44), la valve (44) pouvant être commandée par le dispositif de commutation (57).

8. Dispositif adaptatif selon l'une des revendications 1 à 7, **caractérisé en ce qu'**un module retardateur est prévu permettant de contrôler l'activation du dispositif de commutation (57).

9. Dispositif adaptatif selon la revendication 8, **caractérisé en ce que** le module retardateur est programmable.

10. Dispositif adaptatif selon la revendication 8 ou 9, **caractérisé en ce que** le module retardateur est programmable et/ou commandable par télécommande.

11. Dispositif adaptatif selon l'une des revendications 1 à 10, **caractérisé en ce que** le dispositif adaptatif (10) est réalisé essentiellement en matière plastique.
